(19) Europäisches Patentamt
European Patent Office
Office européen des brevets

(11) Numéro de publication: **0 081 418**
**A1**

(12) # DEMANDE DE BREVET EUROPEEN

(21) Numéro de dépôt: 82402171.1

(22) Date de dépôt: 29.11.82

(51) Int. Cl.³: **C 07 D 471/16**
A 61 K 31/55
//(C07D471/16, 223/00, 221/00, 209/00)

(30) Priorité: 03.12.81 FR 8122642

(43) Date de publication de la demande: 15.06.83 Bulletin 83/24

(84) Etats contractants désignés: AT BE CH DE FR GB IT LI LU NL SE

(71) Demandeur: SYNTHELABO
58, rue de la Glacière
F-75621 Paris Cedex 13(FR)

(72) Inventeur: Bertin, Jean
55, rue d'Estienne d'Orves
F-92140 Clamart(FR)

(72) Inventeur: Frost, Jonathan
15, rue des Amandiers
F-94230 Cachan(FR)

(74) Mandataire: Ludwig, Jacques et al,
SYNTHELABO Service Brevets 58, rue de la Glacière
F-75621 Paris Cedex 13(FR)

(54) **Dérivés oxygènes de diaza-3,7a cyclohepta(j,k)fluorènes, leur préparation et leur application en thérapeutique.**

(57) Composés de formule

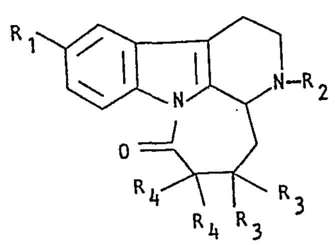

dans laquelle
$R_1$=H, Cl ou $OCH_3$,
$R_2$=H, $CH_3$ ou $CH_2C_6H_5$,
$R_3$=$R_4$=H, ou bien $R_3$=$CH_3$ et $R_4$=H ou bien $R_3$=H et $R_4$=$CH_3$,
leur préparation ainsi que leur application en thérapeutique comme antianoxiques.

EP 0 081 418 A1

0081418

DERIVES OXYGENES DE DIAZA-3,7a CYCLOHEPTA[J,K]FLUORENES, LEUR PREPARATION ET LEUR APPLICATION EN THERAPEUTIQUE.

La présente invention concerne des dérivés oxygénés de diaza-3,7a cyclohepta [j,k] fluorenes, leur préparation et leur application en thérapeutique.

Les composés de l'invention répondent à la formule (I)

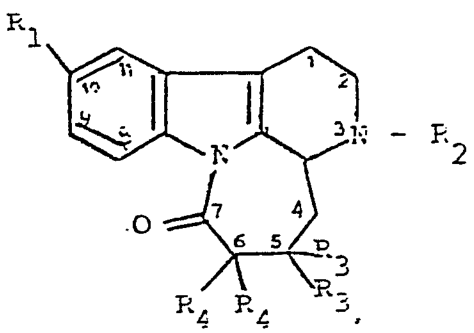

(±)

(I)

dans laquelle

$R_1$ représente un atome d'hydrogène ou de chlore ou un radical méthoxy,

$R_2$ représente un atome d'hydrogène, le radical méthyle ou benzyle,

et soit $R_3$ et $R_4$ représentent des atomes d'hydrogène,

soit $R_3$ est $CH_3$ et $R_4$ est H,

soit $R_3$ est H et $R_4$ est $CH_3$,

à l'exception des composés pour lesquels $R_1$, $R_3$ et $R_4$ représentent chacun, simultanément, un atome d'hydrogène.

Des composés dans la formule desquels $R_1$, $R_3$ et $R_4$ représentent chacun un atome d'hydrogène sont décrits dans le brevet français n° 2 442 236.

Les composés de l'invention peuvent exister sous la forme de racémates ou d'énantiomères. Les sels d'addition qu'ils peuvent former avec des acides acceptables en pharmacologie font également partie de l'invention.

Selon l'invention, on peut préparer les composés (I) selon le schéma réactionnel suivant :

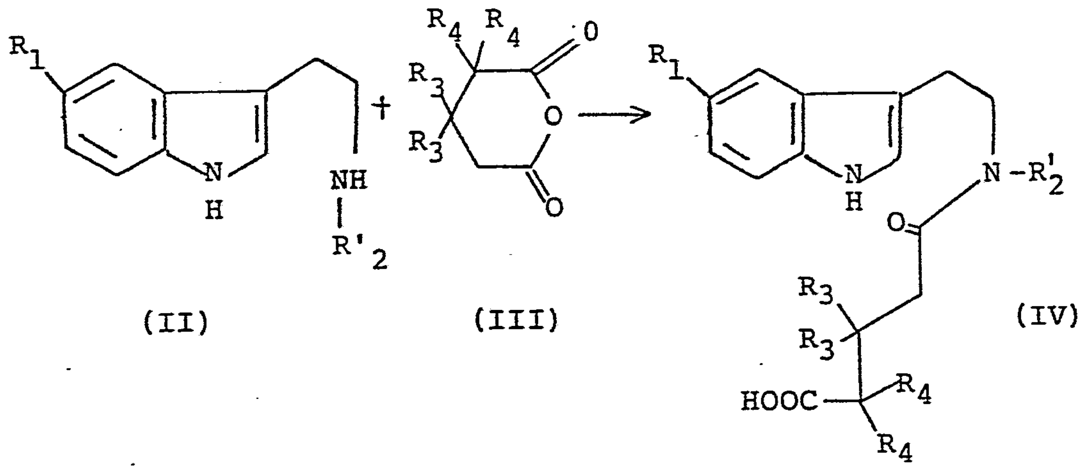

(II) (III) (IV)

$R'_2 = CH_3$ ou $C_6H_5CH_2$

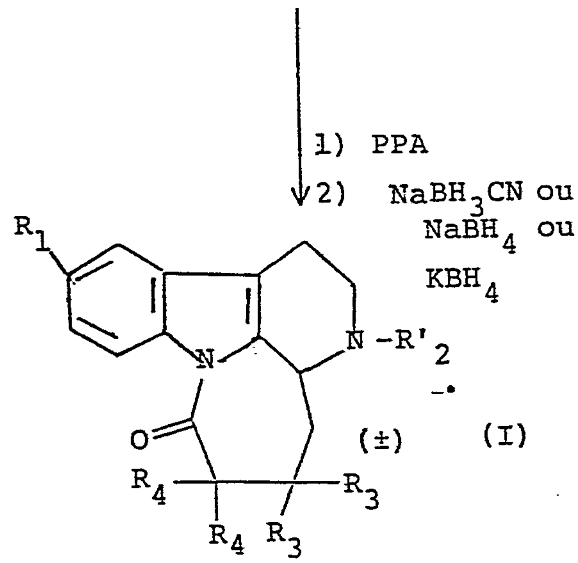


(±) (I)

On fait réagir une tryptamine (II) dans laquelle $R'_2$ est le radical méthyle ou benzyle avec un composé (III) dans un solvant tel que le benzène, à la température du reflux ; puis on fait réagir le composé (IV) à chaud avec de l'acide polyphosphorique puis avec du borohydrocyanure de sodium, ou du borohydrure de sodium ou de potassium ; on obtient alors un composé (I) dans lequel $R'_2$ est $CH_3$ ou $C_6H_5CH_2$ ; pour obtenir un composé (I) dans lequel $R_2$ est H on débenzyle le composé (I) correspondant, dans lequel $R'_2$ est $C_6H_5CH_2$, par une hydrogénation catalytique.

Les exemples suivants illustrent l'invention. Les analyses et les spectres IR et RMN confirment la structure des composés.

Exemple 1

Benzyl-3 diméthyl-5,5 oxo-7 octahydro-1,2,3,3a,4,5,6,7 diaza-3,7a cyclohepta [j,k] fluorène.

1. [ (N-(carboxy-4 diméthyl-3,3 oxo-1 butyl) N-benzyl amino) -2 éthyl]-3 indole.

Dans un erlenmeyer de 500 ml, muni d'une agitation magnétique on introduit 28,6 g de chlorhydrate de (benzylamino-2 éthyl)-3 indole et 150 ml de soude concentrée. On agite à la température ambiante quelques minutes puis on ajoute 200 ml d'acétate d'éthyle et agite à la température ambiante 30 mn. On décante la phase organique, reprend la phase aqueuse avec de l'acétate d'éthyle, regroupe les phases organiques, sèche sur $MgSO_4$ et concentre. On obtient une huile qui est la base de départ (II).

Dans un ballon de 1 l muni d'un réfrigérant, d'une garde de $CaCl_2$, et d'une agitation magnétique, on introduit 25,1 g de la base obtenue ci-dessus dans 200 ml de benzène, puis 14,25 g d'anhydride diméthyl-3,3 glutarique.

On porte le mélange réactionnel à la température du reflux, à l'aide d'un bain d'huile, pendant 2h30, puis on le laisse reposer à la température ambiante pendant une nuit, on concentre et fait cristalliser le composé par addition de 150 ml d'éther isopropylique et agitation. On filtre le produit et le sèche.

F = 124°C

2. Benzyl-3 diméthyl-5,5 oxo-7 octahydro-1,2,3,3a,4,5,6,7 diaza-3,7a cyclohepta [j,k] fluorène et son maléate.

Dans un ballon de 500 ml on introduit 8,95 g du produit brut obtenu précédemment et 65,7 g d'acide polyphosphorique.

On plonge le ballon dans un bain d'huile à 90°C, agite pendant 2h30 et après refroidissement on ajoute 100 ml d'eau, de la soude 5N jusqu'à pH 4 à 5 et on ajoute alors rapidement 4,6 g de $NaBH_3CN$.

On agite le mélange réactionnel à la température ambiante pendant 1 h. On le laisse reposer une nuit à la température ambiante. On ajoute 20 ml d'acide chlorhydrique concentré et on porte le mélange réactionnel à la température de reflux pendant 1 h. On alcalinise après refroidissement avec de la soude 5N puis extrait deux fois avec de l'acétate d'éthyle (400 ml). On sèche sur $MgSO_4$ et concentre. On chromatographie sur 230 g de silice et on élue par $CH_2Cl_2$. A partir de la base ainsi obtenue on prépare le maléate dans de l'acétate d'éthyle. Le maléate, recristallisé dans l'alcool isopropylique, fond à 176-178°C.

<u>Exemple 2</u>

Méthyl-3 diméthyl-6,6 oxo-7 octahydro-1,2,3,3a,4,5,6,7 diaza-3,7a cyclohepta [j,k] fluorène.

1. [ (N-(carboxy-4 diméthyl-4,4 oxo-1 butyl) N-méthyl amino)-2 éthyl] -3 indole.

Dans un ballon de 250 ml muni d'un réfrigérant, d'une garde à $CaCl_2$ et d'une agitation magnétique, on introduit, à la température ambiante, 6,97 g de (méthylamino-2 éthyl)-3 indole, 100 ml de benzène sec et 5,69 g d'anhydride diméthyl-2,2 glutarique.

On porte le mélange réactionnel lentement à la température du reflux, en agitant, pendant 1h30. Puis on le laisse refroidir et reposer une nuit à la température ambiante.

On filtre les cristaux formés, les lave avec du benzène, les sèche et les fait recristalliser dans de l'acétate d'éthyle.

F = 135°C

2. Méthyl-3 diméthyl-6,6 oxo-7 octahydro-1,2,3,3a,4,5,6,7 diaza-3,7a cyclohepta [j,k] fluorène.

Dans un réacteur de 2 l muni d'une agitation mécanique, on introduit 320 g d'acide polyphosphorique et 32 g du composé obtenu précédemment.

On plonge le ballon dans un bain d'huile à 90°C puis agite le mélange réactionnel pendant 3h30. Après refroidissement dans un bain de glace, on ajoute 200 ml d'eau et de la soude 5N jusqu'à pH5. On ajoute alors rapidement 32 g de $NaBH_3CN$ et agite pendant 1 h à la température ambiante. On ajoute lentement 20 ml d'acide chlorhydrique concentré, laisse quelques minutes sous agitation puis ajoute 500 ml d'acétate d'éthyle.

On agite le mélange réactionnel puis le laisse reposer une nuit à la température ambiante. On basifie par de la soude 5N puis on décante la phase organique, reprend la phase aqueuse avec de l'acétate d'éthyle, groupe les phases organiques, sèche sur $MgSO_4$ et concentre.

Après chromatographie sur silice et élution par $CH_2Cl_2$, on obtient une huile.

Exemple 3

Diméthyl-5,5 oxo-7 octahydro-1,2,3,3a,4,5,6,7 diaza-3,7a cyclohepta [j,k] fluorène et son maléate.

Dans un flacon de Parr on place 1 g du produit (base) préparé selon l'exemple 1, avec 25 ml d'éthanol, 2,5 ml d'acide acétique et 0,1 g de charbon palladié à 10 %. On agite la suspension, on établit une pression d'hydrogène d'environ 0,35 MPa et on continue l'agitation pendant 2h30 à température ambiante. Une chromatographie sur couche mince révèle que la réaction est achevée. On ajoute alors quelques gouttes de chloroforme au mélange pour désactiver le catalyseur, on filtre pour éliminer ce dernier, et on concentre le filtrat. On ajoute de l'eau et de l'ammoniaque dilué jusqu'à pH = 10. On extrait deux fois par de l'acétate d'éthyle, on sèche la phase organique sur sulfate de magnésium, puis on la concentre sous vide. On obtient des cristaux blancs.

Le maléate, obtenu par addition d'acide maléique dans une solution de la base (dans l'acétate d'éthyle), est recristallisé dans l'alcool isopropylique. Il fond à 224°C.

Exemple 4

Benzyl-3 chloro-10 oxo-7 octahydro-1,2,3,3a,4,5,6,7 diaza-3,7a cyclohepta [j,k] fluorène.

1. [ (N (carboxy-4 oxo-1 butyl) N benzyl amino)-2 éthyl] -3 indole.

On met en solution 113 g (0,396 mole) de (benzylamino-2 éthyl)-3 chloro-5 indole dans 500 ml de benzène. On ajoute par petites quantités, 45,27 g d'anhydride glutarique, on

porte le mélange réactionnel à la température du reflux pendant 2h puis on le laisse une nuit au repos. On évapore à sec. On obtient le produit.

2. Benzyl-3 chloro-10 oxo-7 octahydro-1,2,3,3a,4,5,6,7 diaza-3,7a cyclohepta [j,k] fluorène et son méthanesulfonate.

On chauffe à 90°C pendant 2h30 un mélange de 20g du composé obtenu précédemment et de 200 g d'acide polyphosphorique. On refroidit le mélange réactionnel et ajoute de la glace et de l'eau. Puis on ajoute de la soude jusqu'à pH4,5, puis on ajoute 18,8 g de $NaBH_3CN$.

On maintient le mélange réactionnel sous agitation pendant 1h30. On refroidit et ajoute de la soude jusqu'à pH7,5. On extrait le produit avec de l'acétate d'éthyle, sèche sur $MgSO_4$ et évapore à sec.

Après chromatographie sur silice et élution avec un mélange 99/1 chloroforme/acétone, on obtient le produit sous forme de base.

On prépare le méthanesulfonate de la base dans de l'éthanol. Le sel est recristallisé dans de l'alcool absolu.

F = 184°C

Exemple 5

Chloro-10 oxo-7 octahydro-1,2,3,3a,4,5,6,7 diaza-3,7a cyclohepta [j,k] fluorène et son chlorhydrate.

On met en solution 3,6 g du composé (base), obtenu selon l'exemple précédent dans 100 ml d'acide acétique et on procède à la débenzylation par l'hydrogène, à température ambiante sous une pression d'environ 0,35 MPa, en présence de 0,36 g

d'oxyde de platine. Il se forme un précipité blanchâtre que l'on dissout par addition d'eau distillée. On élimine le catalyseur par filtration et on évapore le mélange à sec. On reprend par de l'eau ammoniaquée l'huile mi-cristallisée résiduelle, et on extrait la base ainsi libérée par de l'acétate d'éthyle. Les phases organiques réunies, lavées à l'eau, séchées sur sulfate de magnésium, sont évaporées à sec. On chromatographie l'huile obtenue avec un mélange 80/20 de chloroforme et de méthanol. On dissout la base obtenue dans l'éthanol et, en ajoutant de l'éther chlorhydrique 2,5N, on fait précipiter le chlorhydrate pur. On l'isole et le recristallise dans le méthanol. Il ne fond pas en dessous de 300°C.

En repassant à la base, et en chromatographiant celle-ci sur silice avec un mélange 90/10 de chloroforme et de méthanol, on recueille une huile pure qui cristallise dans le pentane.

F = 136-138°C

Le tableau ci-après illustre d'autres composés conformes à l'invention.

TABLEAU

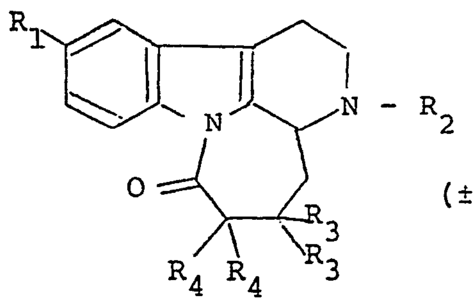

(±)

(I)

| Composé | $R_1$ | $R_2$ | $R_3$ | $R_4$ | Forme | F(°C) |
|---|---|---|---|---|---|---|
| 1 | H | $C_6H_5CH_2$ | $CH_3$ | H | HCCOOH ‖ HCCOOH | 176-8 |
| 2 | H | $CH_3$ | H | $CH_3$ | base | huile |
| 3 | H | H | $CH_3$ | H | HCCOOH ‖ HCCOOH | 224 |
| 4 | Cl | $C_6H_5CH_2$ | H | H | $CH_3SO_3H$ | 184 |
| 5 | Cl | H | H | H | base<br>HCl | 136-8<br>>300 |
| 6 | H | $CH_3$ | $CH_3$ | H | HCCOOH ‖ HCCOOH | 178-80 |
| 7 | H | H | H | $CH_3$ | HCCOOH ‖ HCCOOH | 205 |
| 8 | $CH_3O$ | $C_6H_5CH_2$ | H | H | base<br>HCl | 132-4<br>226-8 |
| 9 | H | $C_6H_5CH_2$ | H | $CH_3$ | HCCOOH ‖ HCCOOH | 164-5 |

Les composés de l'invention ont été soumis à des essais pharmacologiques destinés à mettre en évidence leur intérêt en thérapeutique.

Toxicité aiguë chez la souris

Les composés sont administrés à doses croissantes aux animaux d'essai. La toxicité des composés est exprimée par la dose, en mg par kg de poids corporel, qui laisse vivants la moitié des animaux du lot correspondant à chaque essai. On constate ainsi que, par la voie intrapéritonéale, les $DL_{50}$ des composés s'échelonnent de 30mg/kg à 600 mg/kg, tandis que par voie orale elles vont de 300 mg/kg à plus de 1000 mg/kg.

Activité antianoxique

Administrés par voie intrapéritonéale, les composés de l'invention prolongent la vie des souris placées en atmosphère appauvrie en oxygène (réalisation d'un vide partiel dans une enceinte close où la pression est amenée à $2,5.10^4$ Pa (190 mm Hg) en 30 secondes à l'aide d'une pompe à vide).

L'activité des composés est exprimée par la $DA_{100}$, dose en mg/kg qui prolonge de 100 % la durée de vie des animaux traités, comparée à la durée de vie des animaux témoins.

Les $DA_{100}$ des composés de l'invention se situent entre 5 et 30 mg/kg.

Test de l'ischémie globale chez la souris

On mesure le temps de survie des animaux d'essai après leur avoir injecté, dans la veine caudale, 0,1 ml d'une solution saturée de chlorure de magnésium. L'arrêt cardiaque qui en résulte provoque l'ischémie cérébrale. Le "temps de survie" est l'intervalle de temps entre l'injection du chlorure de magnésium et le dernier mouvement inspiratoire de chaque souris, considéré comme l'indice ultime d'une fonction du

système nerveux central.

On compare les temps de survie des animaux traités par les composés de l'invention, administrés par voie intrapéritonéale 10 minutes avant l'injection du chlorure de magnésium, et les temps de survie des animaux témoins auxquels on n'a administré que le véhicule des substances actives.

Les souris étant étudiées par groupe de 10, les moyennes des résultats de chaque groupe permettent de tracer une courbe, grace à laquelle on détermine graphiquement la Dose Efficace 3, $DE_3$, exprimée en mg de substance active par kg de poids corporel, qui prolonge le temps de survie de 3 secondes.

Une augmentation du temps de survie de 3 secondes est à la fois significative statistiquement et reproductible.

Les $DE_3$ des composés de l'invention vont de 3 à 60 mg/kg.

L'étude pharmacologique des composés de l'invention montre qu'ils possèdent une activité antianoxique et qu'ils peuvent être utilisés en thérapeutique pour le traitement des troubles de la vigilance, en particulier pour lutter contre les troubles du comportement imputables à des dommages vasculaires cérébraux et à la sclérose cérébrale en gériatrie, ainsi que pour le traitement des absences dues à des traumatismes crâniens, pour le traitement des encéphalopathies métaboliques et pour le traitement des états dépressifs.

L'invention comprend par conséquent toutes compositions pharmaceutiques renfermant les composés de l'invention ou leurs sels comme principes actifs, en association avec tous excipients appropriés à leur administration, en particulier par voie orale ou parentérale.

La posologie journalière peut aller de 1 à 100 mg par la voie parentérale, et de 5 à 500 mg par la voie orale, les unités de prise étant par exemple dosées de 1 à 100 mg de substance active.

Revendications pour les états contractants : BE,CH,DE,FR,GB, IT,LI,LU,NL,SE.

1. Composés sous forme de racémates ou d'énantiomères, répondant à la formule (I)

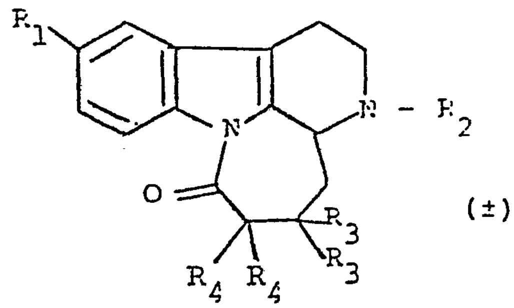

(I)

dans laquelle

$R_1$ représente un atome d'hydrogène ou de chlore ou un radical méthoxy,

$R_2$ représente un atome d'hydrogène, le radical méthyle ou benzyle,

et soit $R_3$ et $R_4$ représentent des atomes d'hydrogène,

soit $R_3$ est $CH_3$ et $R_4$ est H,

soit $R_3$ est H et $R_4$ est $CH_3$,

à l'exception des composés pour lesquels $R_1$, $R_3$ et $R_4$ représentent chacun, simultanément, un atome d'hydrogène,

ainsi que leurs sels d'addition à des acides acceptables en pharmacologie.

2. Procédé de préparation de composés de formule I selon la revendication 1, caractérisé en ce que l'on fait réagir un composé de formule (II)

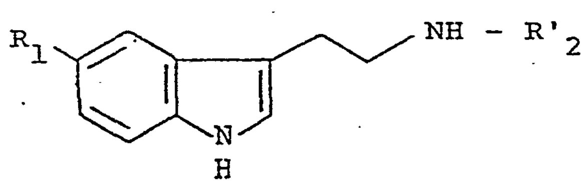

(II)

dans laquelle $R_1$ est tel que défini à la revendication 1 et $R'_2$ représente un radical méthyle ou benzyle,

dans un solvant à la température du reflux,

avec un composé de formule (III)

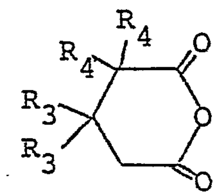

(III)

dans laquelle $R_3$ et $R_4$ sont tels que définis à la revendication 1,

puis on traite le composé obtenu, de formule (IV),

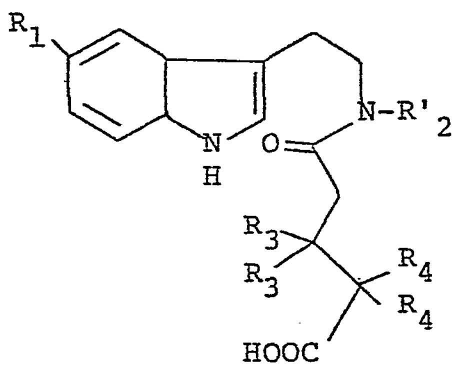

(IV)

d'abord avec de l'acide polyphosphorique puis avec du borohydrure de sodium ou de potassium ou avec du borohydrocyanure de sodium, et, si on désire obtenir un composé de formule I où $R_2$ représente un atome d'hydrogène, on débenzyle le composé de formule I correspondant dans laquelle $R_2$ représente un radical benzyle.

3. Médicament, caractérisé en ce qu'il est constitué par un composé selon la revendication 1

4. Composition pharmaceutique, caractérisée en ce qu'elle contient un composé selon la revendication 1, associé à un excipient acceptable en pharmacologie.

Revendication pour l'état contractant : AT

Procédé de préparation de composés, sous forme de racémates ou d'énantiomères, répondant à la formule (I)

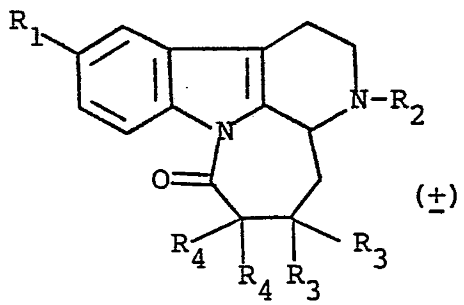

(I)

dans laquelle

$R_1$ représente un atome d'hydrogène ou de chlore ou un radical méthoxy,

$R_2$ représente un atome d'hydrogène ou un radical méthyle ou benzyle,

et, soit $R_3$ et $R_4$ représentent des atomes d'hydrogène,

soit $R_3$ est $CH_3$ et $R_4$ est H,

soit $R_3$ est H et $R_4$ est $CH_3$,

à l'exception des composés pour lesquels $R_1$, $R_3$ et $R_4$ représentent chacun, simultanément, un atome d'hydrogène, ainsi que de leurs sels d'addition à des acides acceptables en pharmacologie,
procédé caractérisé en ce qu'on fait réagir un composé de formule (II)

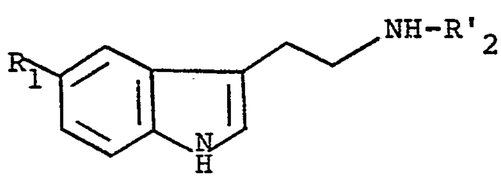

(II)

dans laquelle $R_1$ est tel que défini ci-dessus et $R'_2$ représente un radical méthyle ou benzyle, dans un solvant, à la température du reflux, avec un composé de formule (III)

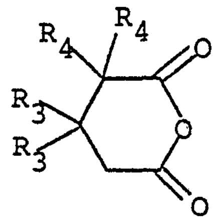

(III)

dans laquelle $R_3$ et $R_4$ sont tels que définis ci-dessus, puis on traite le composé obtenu, de formule (IV)

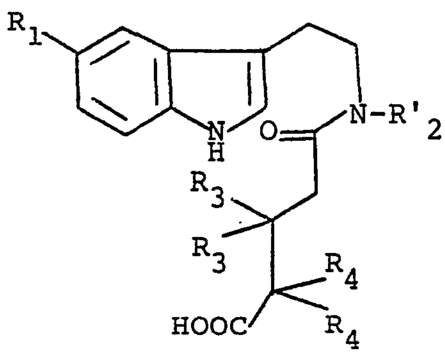

(IV)

d'abord avec de l'acide polyphosphorique puis avec du borohydrure de sodium ou de potassium ou avec du borohydrocyanure de sodium, et, si on désire obtenir un composé de formule I où $R_2$ représente un atome d'hydrogène, on débenzyle le composé de formule I correspondant dans laquelle $R_2$ représente un radical benzyle.

Office européen des brevets

**0081418**

Numéro de la demande

# RAPPORT DE RECHERCHE EUROPEENNE

EP 82 40 2171

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. 3) |
|---|---|---|---|
| D,A | FR-A-2 442 236 (TANABE)<br>*En entier* | 1,3 | C 07 D 471/16<br>A 61 K 31/55 //<br>(C 07 D 471/16<br>C 07 D 223/00<br>C 07 D 221/00<br>C 07 D 209/00 ) |
| | | | **DOMAINES TECHNIQUES RECHERCHES (Int. Cl. 3)** |
| | | | C 07 D 471/00<br>A 61 K 31/00 |

Le présent rapport de recherche a été établi pour toutes les revendications

| Lieu de la recherche | Date d'achèvement de la recherche | Examinateur |
|---|---|---|
| LA HAYE | 25-02-1983 | ALFARO I. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503.03.82